# EUROPÄISCHE PATENTSCHRIFT

(11) **EP 2 359 895 B1**
(45) Veröffentlichungstag und Bekanntmachung des Hinweises auf die Patenterteilung: **03.06.2015**
(21) Anmeldenummer: 11153006.9
(22) Anmeldetag: 02.02.2011
(51) Int. Cl.: A61N 1/05, A61N 1/08, H03H 1/00, H03H 7/01

(54) **Adaptionssonde zur Einführung in implantierte Elektrodenvorrichtungen aktiver medizinischer Implantate sowie Set bestehend aus einer implantierbaren Elektrodenvorrichtung und einer Adaptionssonde**
Adaptation Probe for Insertion into Implanted Electrode Devices of Active Medical Implants and Set Composed of an Implantable Electrode Device and an Adaptation Probe
Sonde d'adaptation pour l'introduction d'implants médicaux actifs dans des dispositifs d'électrodes implantées et ensemble comprenant un dispositif d'électrodes implantables et une sonde d'adaptation

(30) Priorität: 11.02.2010 DE 102010000368; 11.02.2010 DE 102010000370; 11.02.2010 DE 102010000371; 11.02.2010 DE 102010000372
(43) Veröffentlichungstag der Anmeldung: 24.08.2011
(73) Patentinhaber: BIOTRONIK SE & Co. KG, 12359 Berlin (DE)
(72) Erfinder: Weiss, Ingo, 12435 Berlin (DE); Friedrich, Michael, 14532 Kleinmachnow (DE); Knorr, Stefan, 10119 Berlin (DE); Fischer, René, 10247 Berlin (DE); Schurr, Marc Steffen, 10179 Berlin (DE)
(74) Vertreter: Lindner-Vogt, Karin L.

(56) Entgegenhaltungen:
- WO-A1-03/063954
- US-A1- 2004 199 071
- US-A1- 2007 112 398
- US-A1- 2008 116 997
- US-A1- 2009 281 592
- US-A1- 2010 023 000
- US-B2- 6 628 980

## Beschreibung

Die Erfindung betrifft eine Adaptionssonde zur Einführung in implantierte Elektrodenvorrichtungen aktiver medizinischer Implantate zur deren Ertüchtigung zum Einsatz in hochfrequenten magnetischen Wechselfeldern bei MRI-Systemen. Ferner betrifft die Erfindung ein Set bestehend aus einer solchen implantierbaren Elektrodenvorrichtung und einer darin einführbaren Adaptionssonde.

Zum Hindergrund der Erfindung ist festzuhalten, dass der Erfindungsgegenstand in erster Linie im Zusammenhang mit Herzschrittmachern, implantierbaren Defibrillatoren und anderen Typen von aktiven implantierbaren elektromedizinischen Geräten relevant ist. Letztere weisen in aller Regel mindestens eine strom-/spannungsführende Zuleitung in der Elektrodenvorrichtung - üblicherweise als kurz als "Elektrode" bezeichnet - auf, deren distales Ende beispielsweise in einer Herzkammer angeordnet und zur Messung kardiologischer Potentialsignale oder zur Abgabe entsprechender therapeutischer Stromsignale dient.

Die Kompatibilität derartiger Elektrodenvorrichtungen bei implantierbaren elektromedizinischen Geräten mit hochfrequenten Magnetfeldern, wie sie insbesondere bei bildgebenden diagnostischen Verfahren auf Magnetresonanz-Basis - sogenannte MRI- (magnetic resonance imaging) Verfahren - verwendet werden, stellt ein gravierendes Problem dar. Bei solchen MRI-Verfahren wird einem starken statischen Magnetfeld ein mit Radiofrequenz (RF) gepulstes magnetisches Wechselfeld überlagert, das dazu dient, den Energiestatus der Protonen im untersuchten Gewebe zu verändern und entsprechende MRI-Signale aus dem Gewebe zu produzieren.

Dieses magnetische Wechselfeld führt nach den Gesetzen der elektromagnetischen Induktion in den Zuleitung der hier in Rede stehenden Elektrodenvorrichtungen von elektromedizinischen Geräteimplantaten zu Wechselspannungen, deren Energie insbesondere an den elektrisch leitenden Kontaktpolen der Elektrodenvorrichtung zum menschlichen Gewebe in Wärme umgesetzt wird. Dies kann zu einer erheblichen Erhitzung beispielsweise des Spitzenkontaktes einer Herzelektrode mit einer entsprechenden Beeinträchtigung und sogar Schädigung des damit in Berührung stehenden oder umliegenden Herzgewebes führen.

Um diese Problematik zu vermeiden, schlägt die US 7,363,090 B2 die Verwendung von Filtern auf der Basis von Schwingkreisen aus parallel geschalteter Spule und Kondensator vor, die der entsprechenden Zuleitung für den Spitzen-Kontaktpol oder einen Ring-Kontaktpol einer entsprechenden Elektrode eines implantierbaren elektromedizinischen Gerätes zugeordnet ist. Die in diesem vorbekannten Patent offenbarten Filter sind in der Praxisumsetzung der Patentinhaberin als vergleichsweise voluminöse, die Elektrodenvorrichtung auf einer gewissen Länge versteifende Komponenten ausgebildet, die der damit ausgerüsteten Elektrode ungünstige mechanische Eigenschaften verleihen. Ferner ist der Filter in einem in sich geschlossenen Gehäuse untergebracht, das keine Durchführung für die in aller Regel beim Implantieren einer Elektrode verwendeten Führungsdrähte bereit stellt. Insoweit sind die Einsatzmöglichkeiten dieser bekannten Elektrode mit Filtereinrichtung begrenzt.

Aus der US 2009/0281592 A1 sind Filterkomponenten zur Herabsetzung der Erhitzung von Schrittmacher-Elektroden eines elektromedizinischen Implantats bei der Einwirkung hochfrequenter Magnetfelder in Folge von MRI-Prozeduren bekannt, wobei eine Induktionsspule um einen nicht-leitenden, zentralen Abschnitt eines Schaftes aufgebracht ist, der einen Spitzen-Kontaktpol mit einem inneren Wendelleiter der Elektrodenvorrichtung verbindet. Durch die Montage einer Induktionsspule auf dem Schaft wird eine induktive Signalfilterung zur Herabsetzung der Elektrodenspitze ohne die Notwendigkeit eines vergleichsweise langen, voluminösen Spulenkörpers entlang der Länge der Elektrode erreicht. Kapazitive Elemente können ebenfalls in den Schaft integriert werden, um einen LC-Filterkreis zu schaffen. Alternativ dazu ist in dieser Druckschrift eine sogenannte "Luftspule" als induktives Element offenbart, bei der der Schaft weggelassen werden kann.

Von Nachteil bei dem geschilderten Stand der Technik ist die Tatsache, dass derartige Elektrodenvorrichtungen jeweils individuell gemäß ihrer Bauart mit den entsprechenden Schaltungen zur Realisierung einer Filterfunktion bereits bei der Produktion ausgerüstet werden müssen. Herkömmliche Elektrodenvorrichtungen ohne solche Schutzvorrichtungen und insbesondere solche, die bereits ohne die entsprechenden Filtereinrichtungen in einem Patienten implantiert sind, stellen ein Risiko bei Einbringen in ein hochfrequentes magnetisches Wechselfeld dar.

Aus US2010/0023000A1 ist bekannt, nicht replantierte Stimulationsleitungen am proximalen Ende mit einem RF-Filter zu versehen, welches die MR-Frequenzen absorbiert und so verhindert, dass diese im Herzen wirksam werden.

Davon ausgehend liegt der Erfindung die Aufgabe zugrunde, ein System bereitzustellen, mit dessen Hilfe implantierbare Elektrodenvorrichtungen aktiver medizinischer Implantate nachträglich so ertüchtigt werden können, dass sie zum Einsatz in hochfrequenten magnetischen Wechselfeldern insbesondere bei MRI-Systemen geeignet sind.

Diese Aufgabe wird durch die im Patentanspruch 1 definierte Adaptionssonde gelöst, die einen langgestreckten, biegsamen Sondenkörper und eine elektrische Baugruppe mit mindestens einem oder mehreren, zu einer Anpassschaltung verbundenen elektrischen Bauteilen im Sondenkörper aufweist. Diese elektrische Baugruppe ist nach dem Einführen des Sondenkörpers in die vorhandene Elektrodenvorrichtung an eine der Zuleitungen der Elektrodenvorrichtung elektrisch derart ankoppelbar, dass die Elektrodenvorrichtung in ihren elektrischen Eigenschaften, insbesondere in ihrem frequenzabhängigen Widerstand, Impedanz, Kapazität oder Induktivität anpassbar ist. Damit wird eine solche Elektrodenvorrichtung nachträglich so ausgerüstet, dass ein Einsatz in einem hochfrequenten magnetischen Wechselfeld bedenkenlos möglich wird. Einer Erwärmung der Elektrodenvorrichtung, insbesondere deren Kontaktpole, wird durch die Unterbindung entsprechend durch das Wechselfeld induzierter hochfrequenter Ströme in den Zuleitungen der Elektrodenvorrichtung wirkungsvoll vorgebeugt. Dabei erfolgt die Montage der Adaptionssonde in aller Regel erst nach der Implantation der Elektrodenvorrichtung, um deren elektrische Eigenschaften zu verändern. Dies hat den besonderen Vorteil, dass die eigentliche Elektrodenvorrichtung durch die Filterelemente nicht lokal versteift wird. Die Elektrodenvorrichtung ist damit besonders schonend implantierbar. Ihr in aller Regel für einen Führungsdraht vorgesehenes Lumen kann dann verwendet werden, um die Adaptionssonde darin einzuführen.

Gemäß einer bevorzugten Ausführungsform kann der Sondenkörper durch einen isolierten Draht oder einen Kunststoffstab, der gegebenenfalls mit einer Drahtseele versehen ist, gebildet sein. Auf diesen Sondenkörper werden dann elektronische Bauteile aufgebracht und so montiert, dass sie an der funktional gewünschten Längsposition der Adaptionssonde eine elektronische Baugruppe bilden. Diese kann beispielsweise vor der Spitze der Adaptionssonde und/oder an mindestens zwei, vorzugsweise mehreren Längspositionen des Sondenkörpers in diesen integriert sein. Die Spitze des Sondenkörpers kann dabei elektrisch leitend oder auch isolierend ausgebildet sein.

Gemäß einer bevorzugten Ausführungsform ist jede der elektrischen Baugruppen jeweils über einen oder mehrere Anschlusskontakte an die Elektrodenvorrichtung ankoppelbar. Dies kann ein direkter elektrischer Kontakt sein, auch eine kapazitive oder induktive Ankopplung an die Zuleitung oder entsprechende Bauelemente der Elektrodenvorrichtung ist denkbar.

Für den Kontaktschluss ergeben sich verschiedene Konzepte, beispielsweise kann der Anschlusskontakt formschlüssig an eine Zuleitung der Elektrodenvorrichtung anschließbar oder auch als Kontaktfeder, Schleifkontakt oder ähnliche Kontaktfahne geometrisch und körperlich ausgebildet sein. Für eine nachträglich Ertüchtigung einer implantierten Elektrodenvorrichtung ist eine bevorzugte Ausführungsform besonders vorteilhaft, bei der eine oder mehrere elektrische Baugruppen lösbar am Sondenkörper, insbesondere an dessen Spitze befestigt sind. Damit kann nach dem Einführen der Adaptionssonde mit der(den) elektrischen Baugruppe(n) in die Elektrodenvorrichtung und dem Herstellen des geeigneten Kontaktes mit deren Zuleitung die elektrische Baugruppe "abgeworfen" werden. Insoweit muss der eigentliche Sondenkörper nicht in der Elektrodenvorrichtung verbleiben. Es bleibt lediglich die die elektrischen Eigenschaften der Elektrodenvorrichtung anpassende Baugruppe permanent vor Ort. Bei einer entsprechenden Ausgestaltung der Verbindung zwischen elektrischer Baugruppe und Sondenkörper, beispielsweise in Form eines Bajonettverschlusses kann die elektrische Baugruppe auch wieder aus der Elektrodenvorrichtung entfernt werden, indem der Sondenkörper wieder eingeführt wird und die elektrische Baugruppe daran befestigt wird.

Weitere bevorzugte Ausführungsformen betreffen die Ausgestaltung der eigentlichen elektrischen Baugruppe, die beispielsweise elektrische Kontaktpins mit dazwischen geschalteten elektronischen Miniatur-Baulelementen aufweisen kann. Kontaktpins und Miniatur-Bauelemente sind dabei gemeinsam stoffschlüssig in einem umspritzten Filtergehäuse angeordnet. Eine andere Ausführungsform sieht als elektrische Baugruppe einen so zu bezeichnenden "Tonnenfilter" vor, bei dem die Kontaktpins der elektrischen Baugruppe als einander zugewandte, voneinander isolierte Kappen ausgebildet sind, in deren Innenraum die elektrischen Komponenten der elektrischen Baugruppe angeordnet sind.

Da die Adaptionssonde in verschiedene Elektrodenvorrichtungen eingesetzt werden kann, ist es besonders vorteilhaft, wenn die elektrische Baugruppe durch seriell und/oder parallel verschaltete Filterelemente individuell abstimmbar ist, indem diese durch aufbrechbare Kurzschlussleitungen überbrückt sind. Durch das Aufbrechen einer jeweiligen Leitung wird das überbrückte Element aktiviert.

Die Erfindung bezieht sich schließlich auf ein Set, bestehend aus einer implantierbaren Elektrodenvorrichtung eines medizinischen Implantats und einer darin einführbaren Adaptionssonde, die gemäß einer der vorstehend erörterten Ausführungsformen konstruiert ist.

Zusammenfassend stellt die erfindungsgemäße Adaptionssonde eine besonders einfache Lösung für die Ertüchtigung üblicher Elektrodenvorrichtungen von aktiven medizinischen Implantaten zur Verwendung in hochfrequenten magnetischen Wechselfeldern dar. Die eigentliche Konstruktion der Elektrodenvorrichtung braucht nicht geändert zu werden. Die Adaptionssonde stellt eine universelle Lösung für verschiedene Elektrodentypen dar. Gleichwohl wird einer Erwärmung der Elektrodenvorrichtung zuverlässig vorgebeugt, eine Schädigung des Herzmuskels durch eine Erwärmung der Kontaktpole der Elektrodenvorrichtung im MR-Umfeld findet nicht statt. Schließlich besteht ein weiterer Vorteil der Adaptionssonde darin, dass ein bisher ungenutzter Raum, nämlich das eigentlich nur für die Aufnahme des Führungsdrahtes bei der Implantierung der Elektrodenvorrichtung vorgesehene Lumen zur Aufnahme der elektrischen Baugruppe genutzt wird.

Weitere Merkmale, Einzelheiten und Vorteile der Erfindung ergeben sich aus der nachfolgenden Beschreibung von Ausführungsbeispielen anhand der beigefügten Zeichnungen. Es zeigen:
- Fig. 1 bis 3: ausschnittsweise schematische Seitenansichten einer in eine Elektrodenvorrichtung einzuführenden Adaptionssonde in unterschiedlichen Ausführungsformen,
- Fig. 4: eine Adaptionssonde in einem in eine Elektrodenvorrichtung eingeführten Zustand,
- Fig. 5: ein Schaltbild einer elektrischen Baugruppe mit abstimmbarem Frequenzverhalten,
- Fig. 6 und 7: Veranschaulichungen der Kontaktierung zwischen einer elektrischen Baugruppe einer Adaptionssonde und der Elektrodenvorrichtung in zwei Varianten,
- Fig. 8 bis 15: höchst schematische Darstellungen von elektrischen Baugruppen in Form von Hochfrequenz-Filtern in verschiedenen Ausführungsformen,
- Fig. 16 und 17: Teildarstellungen von solchen Hochfrequenz-Filtern, und
- Fig. 18 bis 26: schematische koaxiale Längsschnitte von elektrischen Baugruppen mit integrierten Hochfrequenz-Filtern in unterschiedlichen Ausführungsformen.

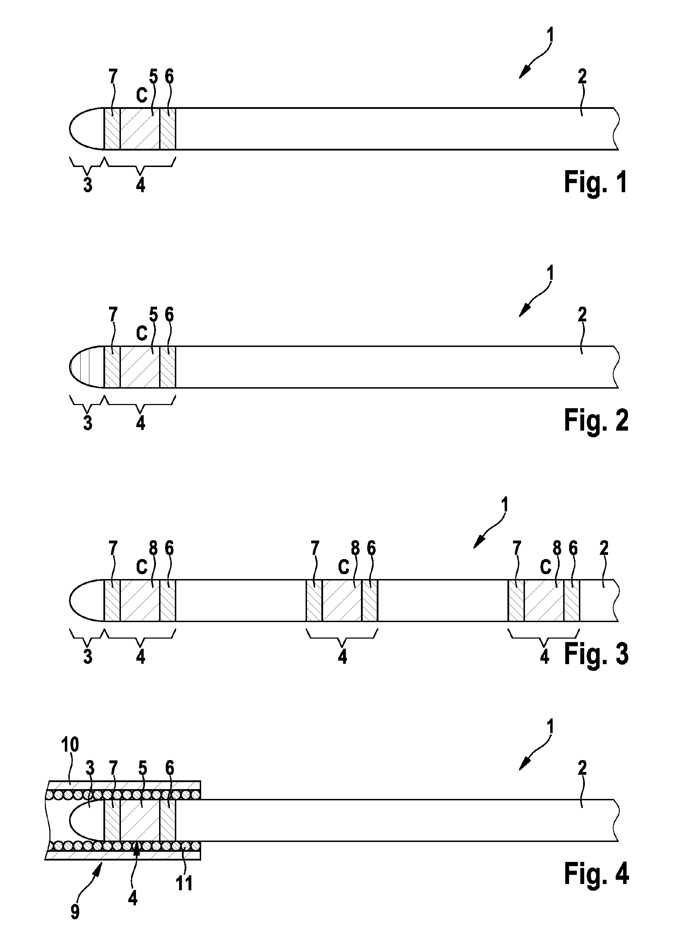

Anhand von Fig. 1 kann die Grundkonfiguration einer auch als "Fertigstellungsdraht" bezeichneten Adaptionssonde 1 näher erläutert werden. So ist ein Sondenkörper 2 aus einem langgestreckten, flexiblen Kunststoff-Stab gebildet, der mit einem (nicht dargestellten) Metallkern versehen ist. Kurz vor der distalen Spitze 3, die in diesem gezeigten Fall ebenfalls nicht leitend ausgebildet ist, ist eine elektrische Baugruppe 4 in den Sondenkörper 1 integriert, die beispielsweise aus einem Kondensator 5 mit einer entsprechenden Kapazität C realisiert sein kann. Dieser Kondensator ist über Anschlusskontakte 6, 7 in noch näher zu erläuternder Weise an eine Zuleitung der Elektrodenvorrichtung elektrisch anzubinden.

Bei der in Fig. 2 gezeigten Ausführungsform ist die Spitze 3 vor der elektrischen Baugruppe 4 leitend ausgebildet.

In Fig. 3 ist eine Adaptionssonde 1 gezeigt, bei der mehrere elektrische Baugruppen 4 mit elektrischen Bauelementen 8, wie beispielsweise wiederum Kondensatoren oder auch komplexere Schaltungen, verteilt über mehrere Längspositionen des Sondenkörpers 2 darin integriert sind.

Fig. 4 zeigt das Grundprinzip der Kooperation zwischen Adaptionssonde 1 und der in dieser Zeichnung ausschnittsweise gezeigten Elektrodenvorrichtung 9. Letztere ist mit ihrem Elektrodenkörper 10 und einer darin zu einem nicht gezeigten Kontaktpol verlaufenden, wendelförmigen Zuleitung 11 dargestellt. Die Adaptionssonde 1 ist in das Lumen 12 der Elektrodenvorrichtung 9 eingeschoben und kontaktiert an geeigneter Stelle mit der wendelförmigen Zuleitung 11. Zwischen den beiden Anschlusskontakten 6, 7 der elektrischen Baugruppe 4 wirkt die Wendelzuleitung als Induktivität und kann zusammen mit dem Kondensator 5 in der Adaptionssonde 1 also einen LC-Schwingkreis mit einem üblichen frequenzabhängigen Übertragungsverhalten bilden. Bei einer entsprechenden Abstimmung werden die in der Zuleitung induzierten Ströme, die aufgrund eines hochfrequenten Wechselfeldes im MR-Umfeld in der Elektrodenvorrichtung entstehen, ausgefiltert.

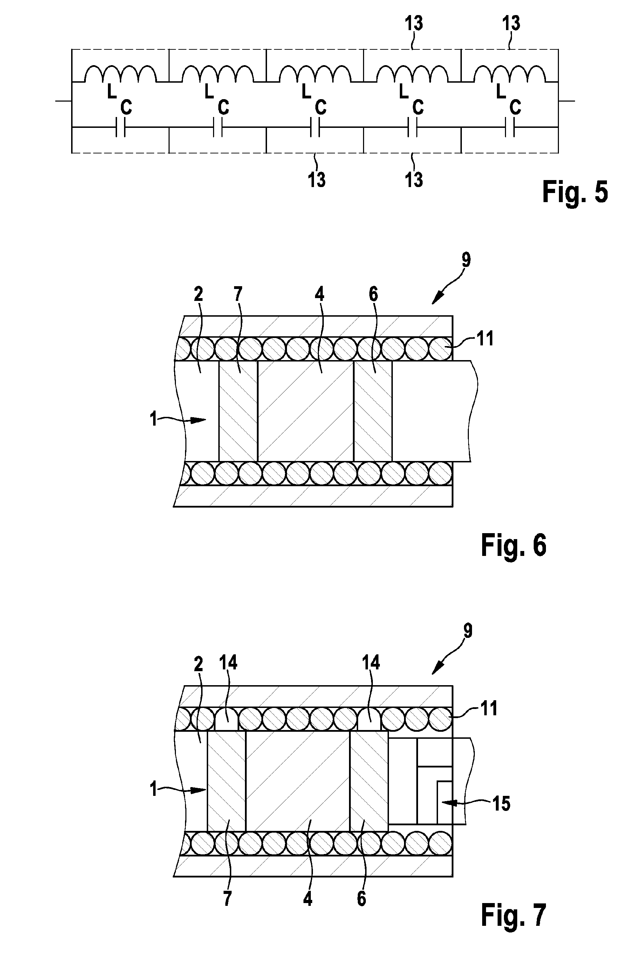

Fig. 5 zeigt eine elektrische Baugruppe in Form eines abstimmbaren Filters, bei dem eine Serienschaltung aus Induktivitäten L parallel zu einer Serienschaltung von Kapazitäten C geschaltet ist. Jede Induktivität L und Kapazität C ist für sich durch Überbrückungsleitungen 13 kurzgeschlossen, die - wie durch strichlierte Linien angedeutet ist - einzeln aufgebrochen werden können. Damit ist die gewünschte Anzahl von Induktivitäten L bzw. Kapazitäten C in den Schwingkreis mit entsprechender Gesamtinduktivität L_{ges} und Gesamtkapazität C_{ges} einzubringen. Auf diese Weise kann eine eventuelle Streuung der Filtereigenschaften während der Produktion ausgeglichen werden. Das sogenannte "Trimmen" erfolgt durch eine Veränderung der Kapazitäten und/oder Induktivitäten.

Anhand der Fig. 6 und 7 sind noch Alternativen bei der Kontaktierung zwischen elektrischer Baugruppe 4 und Zuleitung 11 zu erläutern. So zeigt Fig. 6 eine formschlüssige Kontaktgabe, indem der Sondenkörper 2 mit ringförmigen Anschlusskontakten 6, 7 in das Lumen der Wendelzuleitung 11 eingeschoben und dabei die entsprechende Windung der Wendelzuleitung 11 kontaktiert, die an dieser Stelle abisoliert ist.

Bei der in Fig. 7 gezeigten Ausführungsform sind die Kontakte 6, 7 geometrisch ausgeformt, indem beispielsweise eine seitlich abragende Kontaktfeder 14 die eigentliche elektrische Verbindung zwischen den Anschlusskontakten 6, 7 und der Wendelzuleitung 11 an zwei unterschiedlichen Positionen herstellt.

In Fig. 7 ist auch ein Bajonettverschluss 15 gezeigt, der andeutet, dass in dieser Ausführungsform der distale Endabschnitt der Adaptionssonde 1 in der Elektrodenvorrichtung 9 "abgeworfen" wurde und der verbleibende Sondenkörper 2 wieder entfernt wurde.

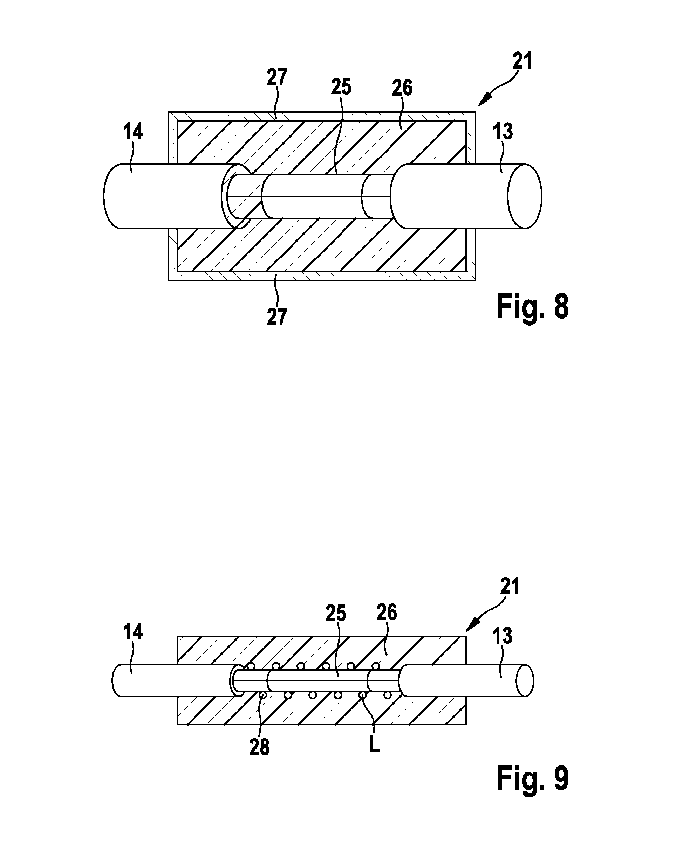

Die Fig. 8 bis 15 zeigen nun unterschiedliche Ausführungsformen, wie elektrische Baugruppen 4 in Form von Hochfrequenz-Filter 21 realisiert werden können, die zum Einführen in eine Elektrodenvorrichtung für einen Herzschrittmacher, Defibrillator, Neurostimulator oder ähnliche aktive medizinische Implantaten geeignet ist.

Gehäuse von vorbekannten Filtern sind in der Regel aus massiven Metallteilen aufgebaut, wobei für den Aufbau einer Isolation zwischen Gehäuse und elektrischen Bauelementen meist teure keramische Bauteile verwendet werden. Die Abdichtung des Gehäuses ist sehr aufwändig, problematisch und damit kostenintensiv. Durch die in den Fig. 8 bis 11 gezeigten Konzepte ist es möglich, eine konstruktiv einfache Abdichtung gegen Flüssigkeiten zu erreichen, so dass der Hochfrequenz-Filter 21 kostengünstig zu realisieren ist. Verschiedenste elektrische Baukomponenten können dabei einfach und variabel eingebettet werden, da das Gehäuse im Wesentlichen durch eine Umspritzung und gegebenenfalls diversen Vor- und Nachbehandlungsschritten entsteht.

Im Detail zeigt Fig. 8 kollinear mit Abstand angeordneten Kontaktpins 23, 24 des Filters 21, zwischen die eine oder mehrere elektrische Bauteile 25 in entsprechender Verschaltung eingebracht und damit verbunden sind. Unter Freilassung der Enden der Kontaktpins 23, 24 wird diese gesamte Baugruppe mit einem Kunststoffkörper 26 umspritzt, der die Abdichtung und elektrische Isolierung der Bauteile 25 gewährleistet.

Bei Bedarf kann der so entstandene Filter 21 noch mit einer Beschichtung 27 versehen werden, die beispielsweise aus einem Kunststoff, einer Keramik oder einer anderweitigen anorganischen Schicht bestehen kann. Eine solche funktionale Beschichtung 27 dient dazu, die Oberflächeneigenschaften an entsprechende Einsatzbedingungen anzupassen, beispielsweise kann die Beschichtung 27 eine mechanische Stabilisierung oder die Ausbildung einer Dampfsperre mit sich bringen.

Die in Fig. 9 dargestellte Ausführungsform unterscheidet sich von der in Fig. 8 dadurch, dass die elektrischen Bauteile 25 zwischen den Kontaktpins 23, 24 noch mit einer Drahtspule 28 umwickelt sind, die die Induktivität L des Hochfrequenz-Filters 21 realisieren kann.

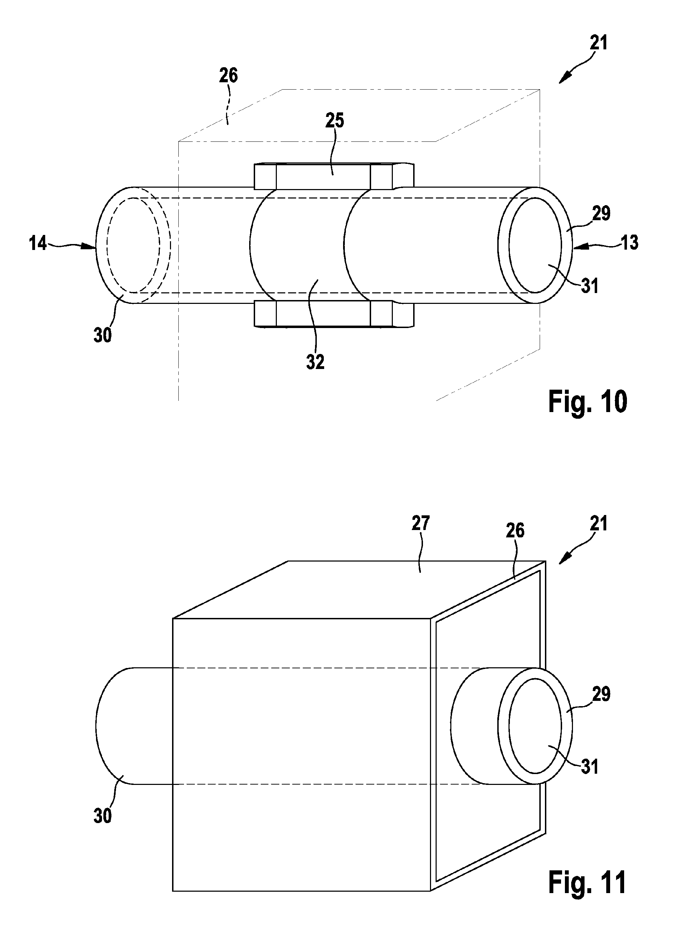

Um eine Adaptionssonde 1 mit einem Hochfrequenz-Filter 21 zu versehen und gleichzeitig die Verwendung eines Führungsdrahtes zu ermöglichen, zeigt Fig. 10 eine Ausführungsform, bei der die Kontaktpins 23, 24 als leitfähige Röhrchen 29, 30 ausgebildet sind, deren Lumen 31 mit einem entsprechenden Durchlass 32 im das Filtergehäuse 22 bildenden Kunststoffkörper 26 fluchtet. Durch Lumen 31 und Durchlass 32 kann dann ein Führungsdraht, Guidewire, Mandrin o. ä. passieren. Wie aus Fig. 10 deutlich wird, sind dabei die elektrischen Bauteile 25 seitlich gegenüber dem Durchlass 32 versetzt eingebettet.

Fig. 11 zeigt nochmals eine Außenansicht des Filters gemäß Fig. 10, wobei auf das Gehäuse wieder eine Beschichtung 27 aus Metall, verschiedenen Kunststoffen bzw. anorganischen oder organischen Verbindungen entsprechend der gewünschten Funktionalität auf gebracht ist.

Die elektrischen Kontaktpins 23, 24 bzw. Röhrchen 29, 30 können aus Edelstahl, Platin, Platin/Iridium-Legierung oder Titan realisiert werden. Auch können sie mit einzelnen oder mehreren Bohrungen, Rillen, Gravierungen oder Vertiefungen versehen sein, um die mechanische Festigkeit des Filters 21 nach der Umspritzung zu erhöhen und diesen insgesamt zu stabilisieren.

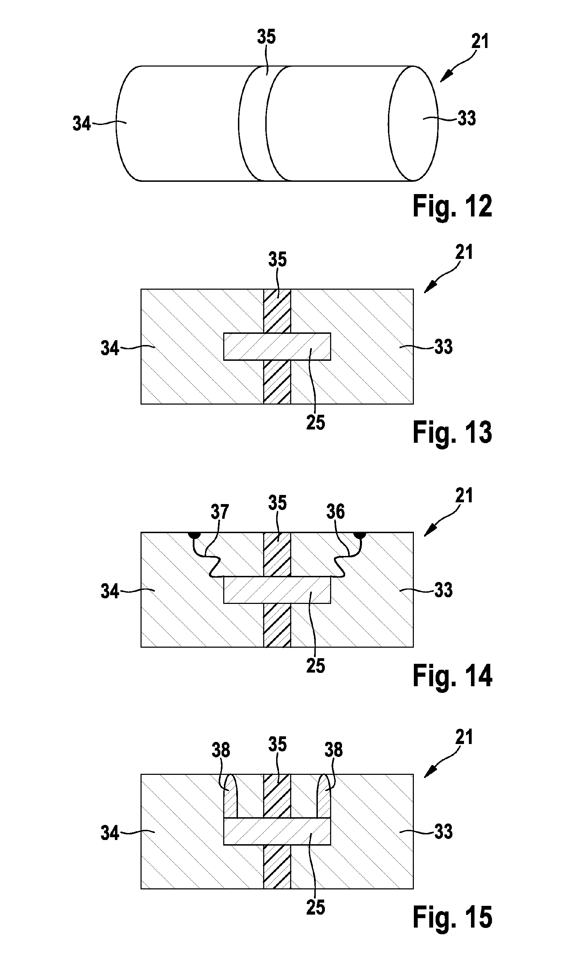

Die folgenden Fig. 12 bis 17 zeigen Ausführungsformen eines Hochfrequenz-Filters 21, die keine Kontaktpins mehr benötigen und deren Gehäuse damit auf einfache Art und Weise abgedichtet werden kann. Bei den vorher beschriebenen Varianten des Filters 21 vergrößern die Kontaktpins 23, 24 die Baugröße des Filters 21, es müssen zusätzliche Passagen isoliert oder abgedichtet werden.

Wie aus der Ansicht gemäß Fig. 12 und dem schematischen Schnitt gemäß Fig. 13 deutlich wird, sind die Kontaktpins durch zwei voneinander isolierte Kontaktkappen 33, 34 gebildet, die über einen Isolatoreinsatz 35 mechanisch verbunden und elektrisch isoliert werden. Die beiden durch die Kontaktkappen 33, 34 gebildeten "Halbtonnen" sind wasserdicht verbunden, es entstehen zwei elektrisch getrennte Bereiche.

In dem Isolatoreinsatz 35 sitzen die elektrischen Bauteile 25 in entsprechender Konfiguration, so dass sie beispielsweise ein Hochpass-, Tiefpass-, Bandpass- oder Bandsperrverhalten aufweisen. Die elektrischen Bauteile 25 werden mit der Innenseite der Kontaktkappen 33 bzw. 34 elektrisch verbunden. Wie in Fig. 14 angedeutet ist, erfolgt dies über entsprechende Anschlussleitungen 36, 37, die durch übliche Drähte, Litzen oder Drahtseile gebildet und in üblicher Weise innen an die Kontaktkappen 33, 34 angeschweißt, gekrimpt oder gelasert sein können. Auch eine induktiv oder kapazitiv koppelnde Verbindung der Anschlüsse ist denkbar.

Die beschriebene Ausbildung des Hochfrequenz-Filters 21 als Tonnenfilter führt zu einer Verkürzung der Bauform und einer Erhöhung der Sicherheit durch eine Reduzierung von Verbindungsstellen. Beim Einbau in eine Adaptionssonde verkürzt sich damit auch der durch den Filter versteifte Bereich.

Wie aus Fig. 15 hervorgeht, kann die elektrische Kontaktierung der Bauteile 25 auch über Schleifkontakte 38 oder entsprechende Kontaktfedern realisiert werden, die mit der Innenseite der Kontaktkappen 33 bzw. 34 elektrisch in Kontakt stehen.

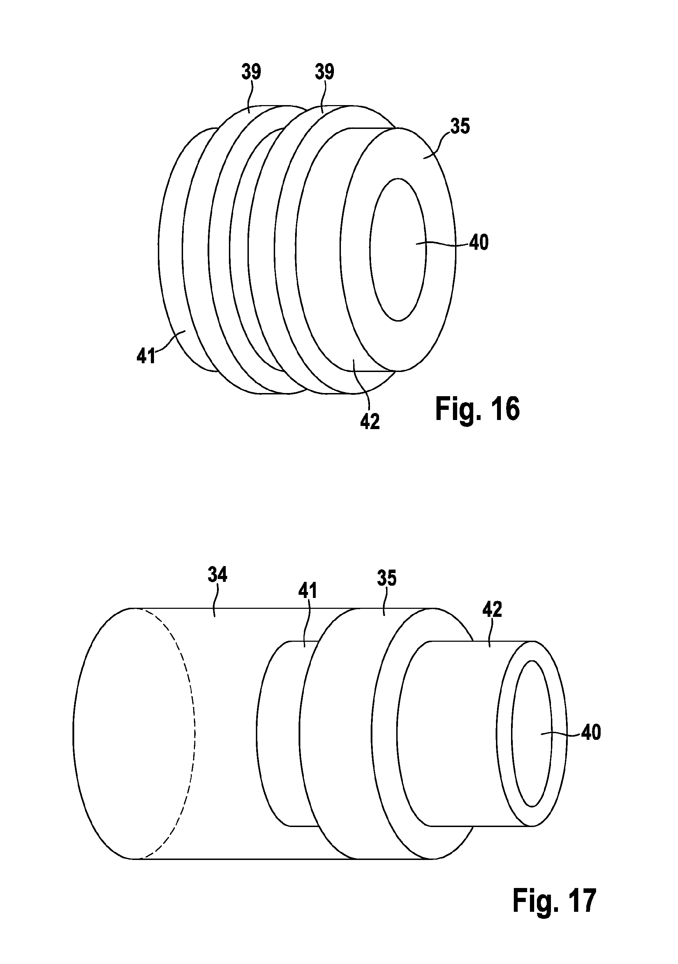

Fig. 16 zeigt eine spezielle Ausbildung des Isolatoreinsatzes 35, der zum einen mit aufgesetzten Schweißscheiben 39 aus Metall versehen ist. Diese stehen radial über die Mantelwand des zylindrisch ausgebildeten Isolatoreinsatzes 35 hinaus und dienen zur Anbindung der Kontaktkappen 33, 34 durch Anschweißen. Ferner weist der Isolatoreinsatz 35 ähnlich einem Rohr koaxial in der Mitte einen Durchgang 40 in Form einer Bohrung oder dergleichen als Aussparung für die Bauteile 25 auf.

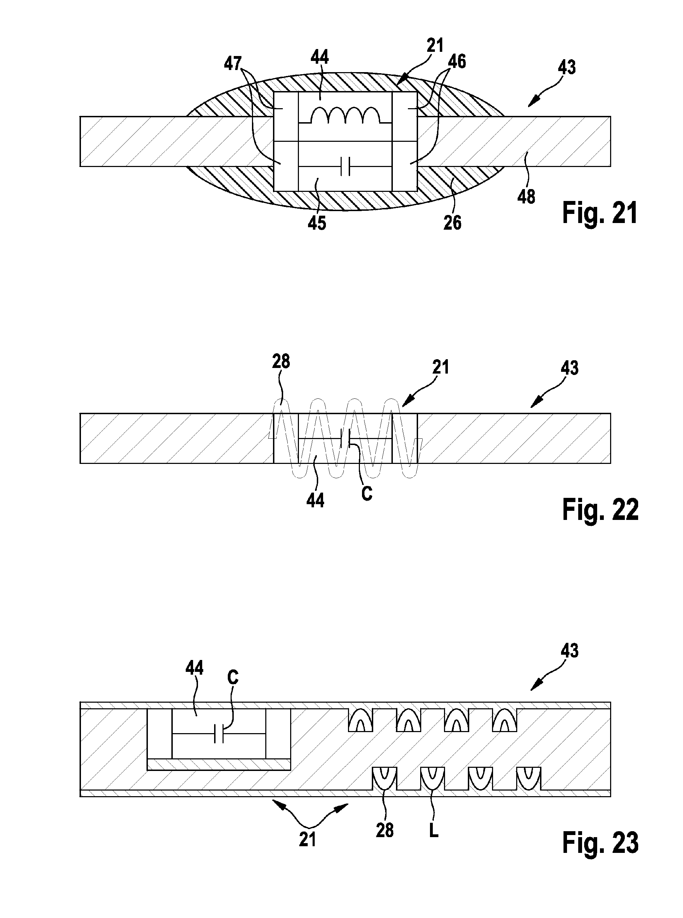

Der Isolatoreinsatz 35 kann als isolierendes Zwischenstück z.B. aus Keramik oder Kunststoff bestehen, auf dessen entsprechende Absätze 41, 42 die Kontaktkappen - in Fig. 21 ist die linke Kontaktkappe 34 gezeigt - aufgeschoben und durch Schweißen, Löten, Kleben, Krimpen oder dergleichen am Isolatoreinsatz 35 befestigt werden können.

Anstatt aus Metall können die zwei Halbtonnen der Kontaktkappen 33, 34 auch aus einem Kunststoff, einem leitfähigen Kunststoff, einer Keramik oder einem anderen Nichtleiter gefertigt werden. Diese müssen dann ganz oder teilweise mit einem leitfähigen Material überzogen sein.

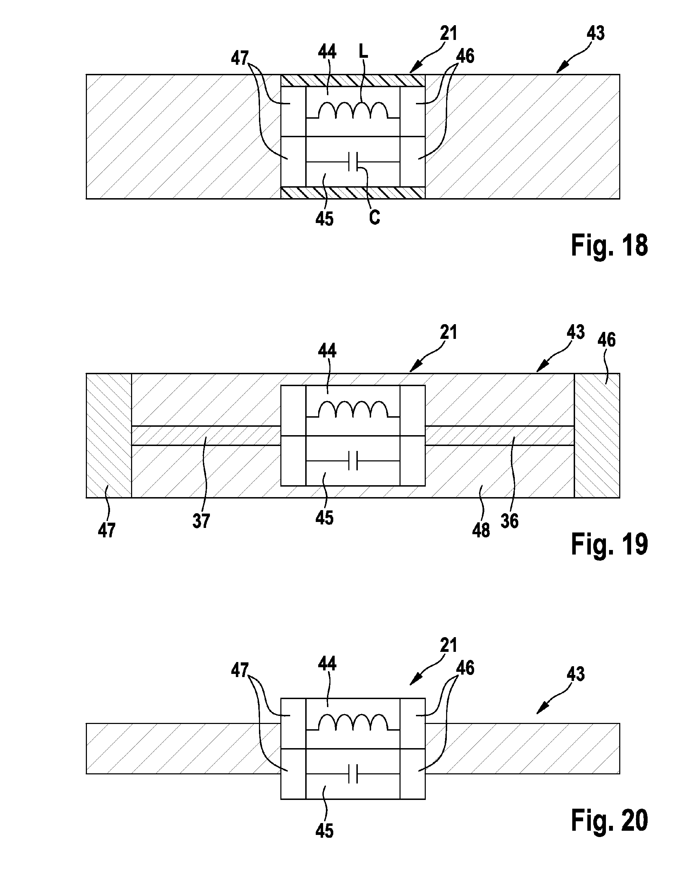

Die Fig. 18 bis 26 schließlich betreffen weitere integrale Bauweisen eines Hochfrequenz-Filters 21.

So zeigt Fig. 18 ein Hochfrequenz-Filterelement 21 als sogenannte Pin-Einheit 43, bei der ein Filter bestehend aus zwei SMD-Bauteilen 44, 45 in Form einer Induktivität L und einer Kapazität C unter deren Parallelverschaltung realisiert ist. Die Bauform der SMD-Bauteile 44, 45 muss nicht gleich sein. Sie sind komplett in die Pin-Einheit integriert, diese kann damit isodiametrisch hergestellt werden. Die Anschlüsse 46, 47 links und rechts bestehen aus leitfähigem Material.

Wie aus Fig. 19 hervorgeht, kann die Pin-Einheit 43 auch aus einem Körper 48 aus dielektrischem Material bestehen, der entsprechende Anschlussleitungen 36, 37 zwischen den die Filterkomponenten realisierenden SMD-Bauteilen 44, 45 und den Anschlüssen 46, 47 aufweist. Diese Bauform beansprucht die Filterkomponenten weniger, da sie in einem homogenen Material eingebettet sind.

Bei der in Fig. 20 gezeigten Ausführungsform ist der Hochfrequenz-Filter 21 mit seinen SMD-Bauteilen 44, 45 in einen Körper 48 mit einer relativ dünnen Struktur integriert. Die Montage zwischen zwei leitfähigen Elementen ist in dieser Zeichnung weggelassen. Die gesamte Pin-Einheit 43 ist dann nicht zwingend isodiametrisch. Wie anhand von Fig. 19 beschrieben ist, kann diese Ausführungsform auch aus dielektrischem Material mit geeigneten Leitungsstrukturen bestehen.

Um den Hochfrequenz-Filter 21 selbst von der Umgebung abzutrennen, wird er - wie Fig. 21 zeigt - mit einem umspritzten Kunststoffkörper 26, einer Beschichtung, einem Gehäuse oder ähnlichen Maßnahmen umhüllt.

Eine weitere Miniaturisierung für die Pin-Einheit 43 wird mit der in Fig. 22 gezeigten Ausführungsform erzielt. Dort ist die Drahtspule 28 des Hochfrequenz-Filters 21 um das als Kapazität C ausgelegte SMD-Bauteil 44 herum angelegt. Damit wird das benötigte Bauvolumen gegenüber den vorher erörterten Ausführungsformen gemäß Fig. 18 bis 21 insbesondere im Durchmesser deutlich verringert.

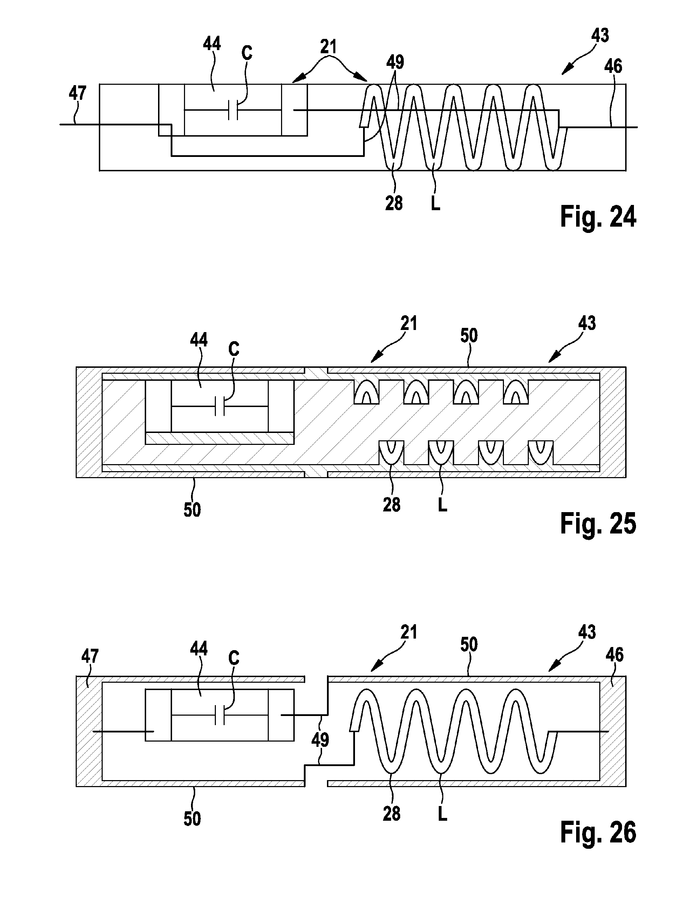

Bei dem in Fig. 24 dargestellten Ausführungsbeispiel der Pin-Einheit 43 werden die Spule 28 und das SMD-Bauteil 44 für die Kapazität C mechanisch hintereinander montiert, wobei die in dicken Linien herausgezeichnete Verschaltung 49 parallel erfolgt. Die Pin-Einheit kann dabei isodiametrisch ausgeführt sein und an ihren Enden die entsprechenden Anschlüsse 46, 47 aufweisen. Die Kapazität C ist als Kondensator-SMD-Bauteil 44 und die Induktivität L als Drahtspule im Inneren des Bauteils montiert. In Fig. 24 ist die tragende Struktur des Körpers der Pin-Einheit der Übersichtlichkeit halber weggelassen.

Eine solche ist in Fig. 25 erkennbar. Ferner weist diese Ausführungsform eine Metallisierung 50 auf, wie sie sich über weite Teilbereiche der Pin-Einheit 43 auf deren Außenseite erstreckt. Über diese Metallisierung 50 erfolgt die Kontaktierung der Bauteile, nämlich der Drahtspule 28 und des Kondensator-SMD-Bauteils 44, wie dies in Fig. 26 erkennbar ist. Die Verschaltung 49 kann damit im Inneren mit kürzeren Wegen auskommen.

**Bezugszeichenliste**

| | | | |
|---|---|---|---|
| 1 | Adaptionssonde | 31 | Lumen |
| 2 | Sondenkörper | 32 | Durchlass |
| 3 | Spitze distal | 33 | Kontaktkappe |
| 4 | elektrische Baugruppe | 34 | Kontaktkappe |
| 5 | Kondensator | 35 | Isolatoreinsatz |
| 6 | Anschlusskontakt | 36 | Anschlussleitung |
| 7 | Anschlusskontakt | 37 | Anschlussleitung |
| 8 | elektrisches Bauelement | 38 | Schleifkontakt |
| 9 | Elektrodenvorrichtung | 39 | Schweißscheibe |
| 10 | Elektrodenkörper | 40 | Durchgang |
| 11 | Wendelzuleitung | 41 | Absatz |
| 12 | Lumen | 42 | Absatz |
| 13 | Überbrückungsleitung | 43 | Pin-Einheit |
| 14 | Kontaktfeder | 44 | SMD-Bauteil |
| 15 | Bajonettverschluss | 45 | SMD-Bauteil |
| 16 | distaler Endabschnitt | 46 | Anschluss |
| 17 | Hochfrequenz-Filter | 47 | Anschluss |
| 18 | Gehäuse | 48 | Körper |
| 19 | Kontaktpin | 49 | Verschaltung |
| 20 | Kontaktpin | 50 | Metallisierung |
| 25 | elektrische Bauteile | | |
| 26 | Kunststoffkörper | | |
| 27 | Beschichtung | | |
| 28 | Drahtspule | | |
| 29 | Röhrchen | | |
| 30 | Röhrchen | | |

## Patentansprüche

1. Adaptionssonde zur Einführung in implantierte Elektrodenvorrichtungen aktiver medizinischer Implantate zu deren Ertüchtigung zum Einsatz in hochfrequenten magnetischen Wechselfeldern bei MRI-Systemen,
**gekennzeichnet durch**
- einen langgestreckten, biegsamen Sondenkörper (2), und
- mindestens eine elektrische Baugruppe (4) mit einem oder mehreren, zu einer Anpass-Schaltung verbundenen elektrischen Bauteilen (8, 25) im Sondenkörper (2), die an eine Zuleitung (11) der Elektrodenvorrichtung (9) elektrisch derart ankoppelbar ist, dass die Elektrodenvorrichtung (9) in ihren elektrischen Eigenschaften, insbesondere in ihrem frequenzabhängigen Widerstand, Impedanz, Kapazität oder Induktivität, anpassbar ist.

2. Adaptionssonde nach Anspruch 1, **dadurch gekennzeichnet, dass** der Sondenkörper (2) durch einen isolierten Draht oder einen Kunststoffstab gegebenenfalls mit einer Drahtseele gebildet ist.

3. Adaptionssonde nach Anspruch 1 oder 2, **dadurch gekennzeichnet, dass** die elektrische Baugruppe (4) vor der Spitze (3) der Adaptionssonde (1) in den Sondenkörper (2) integriert.

4. Adaptionssonde nach einem der vorgenannten Ansprüche, **dadurch gekennzeichnet, dass** elektrische Baugruppen (4) an mindestens zwei, vorzugsweise mehreren Längspositionen des Sondenkörpers (2) in diesen integriert sind.

5. Adaptionssonde nach einem der vorgenannten Ansprüche, **dadurch gekennzeichnet, dass** die Spitze (3) des Sondenkörpers (2) elektrisch leitend oder isolierend ausgebildet ist.

6. Adaptionssonde nach einem der vorgenannten Ansprüche, **dadurch gekennzeichnet, dass** die elektrische(n) Baugruppe(n) (4) jeweils über einen Anschlusskontakt (6, 7; 14; 23, 24; 33, 34) an die Elektrodenvorrichtung ankoppelbar ist (sind).

7. Adaptionssonde nach Anspruch 6, **dadurch gekennzeichnet, dass** der Anschlusskontakt (6, 7; 23, 24; 33, 34) formschlüssig an die Zuleitung der Elektrodenvorrichtung (9) anschließbar ist.

8. Adaptionssonde nach Anspruch 6, **dadurch gekennzeichnet, dass** der Anschlusskontakt als Kontaktfeder (14), Schleifkontakt oder Kontaktfahne ausgebildet ist.

9. Adaptionssonde nach einem der vorgenannten Ansprüche, **dadurch gekennzeichnet, dass** die elektrische(n) Baugruppe(n) (4) lösbar am Sondenkörper (2), insbesondere an dessen Spitze (3) befestigt ist (sind).

10. Adaptionssonde nach Anspruch 9, **dadurch gekennzeichnet, dass** die elektrische(n) Baugruppe(n) (4) vorzugsweise mittels eines Bajonettverschlusses (15) am Sondenkörper (2) lösbar und wieder anbringbar befestigt sind.

11. Adaptionssonde nach einem der vorgenannten Ansprüche, **dadurch gekennzeichnet, dass** die elektrische Baugruppe(n) (4) elektrische Kontaktpins (23, 24) mit dazwischen geschalteten elektronischen Miniatur-Bauelementen (25) aufweist (aufweisen), wobei Kontaktpins (23, 24) und Miniatur-Bauelemente (25) gemeinsam stoffschlüssig in einem umspritzten Filtergehäuse (26) angeordnet sind.

12. Adaptionssonde nach einem der vorgenannten Ansprüche, **dadurch gekennzeichnet, dass** Kontaktpins (23, 24) der elektrischen Baugruppe(n) (4) als einander zugewandte, voneinander isolierte Kontaktkappen (33, 34) ausgebildet sind, in deren Innenraum die elektrischen Komponenten (25) der elektrischen Baugruppe (4) angeordnet sind.

13. Adaptionssonde nach einem der vorgenannten Ansprüche, **dadurch gekennzeichnet, dass** die elektrische Baugruppe (4) seriell und/oder parallel verschaltete Filterelemente (L, C) aufweist, die durch aufbrechbare Kurzschlussleitungen (13) überbrückt sind.

14. Set bestehend aus einer implantierbaren Elektrodenvorrichtung (9) eines aktiven medizinischen Implantats und einer darin einführbaren Adaptionssonde (1) nach einem der vorgenannten Ansprüche.

## Claims

1. An adaptation probe for insertion into implanted electrode devices of active medical implants for retrofitting thereof for use in high-frequency magnetic alternating fields of MRI systems,
**characterised by**
- an elongate, flexible probe body (2), and
- at least one electrical assembly (4) comprising one or more electrical components (8, 25) in the probe body (2), which are connected to a customisation circuit and which can be electrically coupled to a supply lead (11) of the electrode device (9) such that electrical properties of the electrode device (9), in particular the frequency-dependent resistance, impedance, capacitance, or inductance thereof, can be customised.

2. The adaptation probe according to Claim 1, **characterised in that** the probe body (2) is formed by an insulated wire or a plastic rod, where appropriate with a wire core.

3. The adaptation probe according to Claim 1 or 2, **characterised in that** the electrical assembly (4) is integrated into the probe body (2) before the tip (3) of the adaptation probe (1).

4. The adaptation probe according to one of the preceding claims, **characterised in that** electrical assemblies (4) are integrated into the probe body (2) at least at two, preferably more, longitudinal positions thereof.

5. The adaptation probe according to one of the preceding claims, **characterised in that** the tip (3) of the probe body (2) is electrically conductive or insulating.

6. The adaptation probe according to one of the preceding claims, **characterised in that** the electrical assembly/assemblies (4) can be coupled to the electrode device in each case via a connection contact (6, 7; 14; 23, 24; 33, 34).

7. The adaptation probe according to Claim 6, **characterised in that** the connection contact (6, 7; 23, 24; 33, 34) can be connected to the supply lead of the electrode device (9) in a form-fitting manner.

8. The adaptation probe according to Claim 6, **characterised in that** the connection contact is formed as a contact spring (14), sliding contact, or contact tab.

9. The adaptation probe according to one of the preceding claims, **characterised in that** the electrical assembly/assemblies (4) is/are detachably secured to the probe body (2), in particular to the tip (3) thereof.

10. The adaptation probe according to Claim 9, **characterised in that** the electrical assembly/assemblies (4) preferably can be detached from and reattached to the probe body (2) by means of a bayonet connection (15).

11. The adaptation probe according to one of the preceding claims, **characterised in that** the electrical assembly/assemblies (4) comprises/comprise electrical contact pins (23, 24) having miniature electronic components (25) connected therebetween, wherein contact pins (23, 24) and miniature components (25) are arranged jointly in an integrally bonded manner in a filter housing (26) encapsulated by insert moulding.

12. The adaptation probe according to one of the preceding claims, **characterised in that** contact pins (23, 24) of the electrical assembly/assemblies (4) are formed as mutually opposed contact caps (33, 34) that are insulated from one another, in the interior of which the electrical components (25) of the electrical assembly (4) are arranged.

13. The adaptation probe according to one of the preceding claims, **characterised in that** the electrical assembly (4) has series- and/or parallel-connected filter elements (L, C), which are bridged by separable short-circuit lines (13).

14. A set consisting of an implantable electrode device (9) of an active medical implant and an adaptation probe (1) that can be inserted therein, according to one of the preceding claims.

## Revendications

1. Sonde d'adaptation pour l'introduction dans des dispositifs d'électrodes implantées d'implants médicaux actifs, pour développer ceux-ci en vue d'une utilisation dans des champs magnétiques alternatifs à haute fréquence dans des systèmes d'IRM,
**caractérisée par**
- un corps de sonde (2) flexible allongé, et
- au moins un module électrique (4) avec un ou plusieurs composants électriques (8, 25) reliés à un circuit d'adaptation dans le corps de sonde (2), ledit module pouvant être couplé électriquement à une ligne d'arrivée (11) du dispositif d'électrodes (9), de telle façon que le dispositif d'électrodes (9) peut être adapté quant à ses propriétés électriques, en particulier sa résistance en fonction de la fréquence, son impédance, sa capacité ou son inductance.

2. Sonde d'adaptation selon la revendication 1, **caractérisée en ce que** le corps de sonde (2) est formé par un fil isolé ou par une tige en plastique, éventuellement avec une âme métallique.

3. Sonde d'adaptation selon la revendication 1 ou 2, **caractérisée en ce que** le module électrique (4) est intégré dans le corps de sonde (2) en amont de la pointe (3) de la sonde d'adaptation (1).

4. Sonde d'adaptation selon l'une des revendications précédentes, **caractérisée en ce que** des modules électriques (4) sont intégrés dans le corps de sonde (2) en au moins deux positions longitudinales de celui-ci, de préférence plusieurs.

5. Sonde d'adaptation selon l'une des revendications précédentes, **caractérisée en ce que** la pointe (3) du corps de sonde (2) est conçue électriquement conductrice ou isolante.

6. Sonde d'adaptation selon l'une des revendications précédentes, **caractérisée en ce que** le(s) module(s) électrique(s) (4) peut/peuvent être couplé(s) au dispositif d'électrodes respectivement par le biais d'un contact de raccordement (6, 7 ; 14 ; 23, 24 ; 33, 34).

7. Sonde d'adaptation selon la revendication 6, **caractérisée en ce que** le contact de raccordement (6, 7 ; 14 ; 23, 24 ; 33, 34) peut être raccordé par complémentarité de forme à la ligne d'arrivée du dispositif d'électrodes (9).

8. Sonde d'adaptation selon la revendication 6, **caractérisée en ce que** le contact de raccordement est conçu comme un ressort de contact (14), un contact frottant ou un talon de contact.

9. Sonde d'adaptation selon l'une des revendications précédentes, **caractérisée en ce que** le(s) module(s) électrique(s) (4) est/sont fixé(s) de façon détachable au corps de sonde (2), en particulier à la pointe (3) de celui-ci.

10. Sonde d'adaptation selon la revendication 9, **caractérisée en ce que** le(s) module(s) électrique(s) (4) est/sont fixé(s) de manière à pouvoir être détachés et fixés à nouveau au corps de sonde (2), de préférence au moyen d'une fixation à baïonnette (15).

11. Sonde d'adaptation selon l'une des revendications précédentes, **caractérisée en ce que** le(s) module(s) électrique(s) (4) comporte/comportent des broches de contact électriques (23, 24) avec des composants électroniques miniatures (25) raccordés entre celles-ci, les broches de contact (23, 24) et les composants miniatures (25) étant agencés ensemble par coopération de matières dans un boîtier de filtre (26) surmoulé.

12. Sonde d'adaptation selon l'une des revendications précédentes, **caractérisée en ce que** les broches de contact (23, 24) du/des module(s) électrique(s) (4) sont conçues comme des capuchons de contact (33, 34) tournés les uns vers les autres, isolés les uns des autres, dans l'espace intérieur desquels sont agencés les composants électriques (25) du module électrique (4).

13. Sonde d'adaptation selon l'une des revendications précédentes, **caractérisée en ce que** le module électrique (4) comporte des éléments filtrants (L, C) couplés en série et/ou en parallèle, lesquels sont pontés par des lignes de court-circuit (13) susceptibles d'être rompues.

14. Ensemble constitué d'un dispositif d'électrodes (9) implantable d'un implant médical actif et d'une sonde d'adaptation (1) susceptible d'être introduite dans celui-ci, selon l'une des revendications précédentes.
